# EUROPÄISCHE PATENTSCHRIFT

(11) **EP 2 626 050 B1**
(45) Veröffentlichungstag und Bekanntmachung des Hinweises auf die Patenterteilung: **13.04.2016**
(21) Anmeldenummer: 12193540.7
(22) Anmeldetag: 21.11.2012
(51) Int. Cl.: A61F 13/15

(54) **Verfahren und Vorrichtung zur Herstellung eines Laminats und Hygieneprodukts**
Method and device for producing a laminate and hygiene product
Procédé et dispositif de fabrication d'un laminé et d'un produit d'hygiène

(30) Priorität: 16.01.2012 DE 102012200555
(43) Veröffentlichungstag der Anmeldung: 14.08.2013
(73) Patentinhaber: Bikoma GmbH Spezialmaschinen, 56727 Mayen (DE)
(72) Erfinder:
(74) Vertreter: Lemcke, Brommer & Partner Patentanwälte Partnerschaft mbB

(56) Entgegenhaltungen:
- WO-A1-2004/073568
- US-A- 4 227 952
- US-A- 4 364 787
- US-A- 5 380 381
- US-A- 6 165 306
- US-A1- 2002 129 687
- US-A1- 2009 320 663
- US-A1- 2010 078 120

## Beschreibung

Die vorliegende Erfindung betrifft ein Verfahren zur Herstellung eines Laminats nach dem Oberbegriff des Patentanspruchs 1, welches Laminat eine erste Materialbahn umfasst, vorzugsweise aus einem textilen Flächengebilde, höchst vorzugsweise Vlies- oder Nonwoven-Material, oder aus Folie, welche erste Materialbahn mit Abschnitten wenigstens einer zweiten Materialbahn aus einem elastisch dehnbaren Material, vorzugsweise Schaumstoff, verbunden wird.

Weiterhin betrifft die Erfindung eine als Applikatoreinrichtung bezeichnete Vorrichtung zur Herstellung eines Laminats der vorstehend beschriebenen Art, nach dem Oberbegriff des Patentanspruchs 6.

Insbesondere zur Herstellung sogenannter körpergeförmter Inkontinenzprodukte, d.h. windelartiger Hygieneprodukte mit Kreppung insbesondere im Randbereich, um eine dreidimensionale, körpergerechte Produktformung zu erreichen, wurden in der Vergangenheit in relativ aufwändiger Art und Weise Elastan-Fasern (Lycra®) verwendet, um zusammen mit einem (Nonwoven-)Material körpergeformte Hygieneprodukte zu erzeugen. Ein alternativer Ansatz besteht darin, Streifen oder Abschnitte aus einem elastisch dehnbaren Material, z.B. Schaumstoff, im gedehnten Zustand auf das (Nonwoven-)Material aufzukleben, um auf diese Weise eine Kreppung und die gewünschte Formgebung zu erreichen. Dies geschieht nach dem Stand der Technik regelmäßig diskontinuierlich getaktet, so dass die erreichbaren Durchsatzraten entsprechend gering sind.

Im Rahmen der vorliegenden Beschreibung wird die verbundene Anordnung des ersten Materials (des (Nonwoven-)Materials) und des zweiten Materials (z.B. Schaumstoff) als "Laminat" bezeichnet. Damit ist keine Einschränkung dahingehend verbunden, das eine komplett-vollflächige Verbindung bzw. Bedeckung des ersten Materials mit dem zweiten Material erfolgen muss. Die genannte Verbindung kann eine Klebeverbindung, eine (Ultraschall-)Prägeverbindung oder eine andere Verbindung sein. Sie muss nicht vollflächig erfolgen.

Aus der WO 2004/073568 A1 ist eine Anordnung und ein Verfahren zum Anbringen von elastischen Elementen an einer Materialbahn bekannt, wobei verschiedene Walzen der Anordnung genau aufeinander abgestimmte Umfangsgeschwindigkeiten aufweisen. Zur Erzeugung von Artikeln mit geänderten Formaten müssen zumindest einzelne Walzen oder sogar die gesamte Anordnung ausgetauscht werden, was aufwändig und kostspielig ist.

Die US 6,165,306 A offenbart ein Verfahren und eine Vorrichtung zum Zerteilen wenigstens zweier Materialbahnen und zum definierten Anordnen der Teile relativ zueinander auf einer bewegten weiteren Materialbahn.

Die US 5,380,381 betrifft eine Etikettiermaschine mit einem Schneidkopf, der mit variabler Geschwindigkeit betreibbar ist.

US 4,227,952 offenbart eine Vorrichtung zur Herstellung von Windeln mit umlaufenden Förderketten.

US 2010/0078120 A1 offenbart eine Vorrichtung zur Herstellung von Hygieneartikeln mit kontinuierlich zugeführtem elastischem Material.

Ähnliche Vorrichtungen sind aus der US 2009/0320663 A1, der US 2002/0129687 A1 und der US 4,364,787 bekannt.

Der Erfindung liegt die Aufgabe zugrunde, ein Verfahren und eine Vorrichtung der jeweils eingangs definierten Art dahingehend weiterzuentwickeln, dass in einfacher Weise eine Umstellung auf geänderte Formate erfolgen kann.

Die Erfindung löst diese Aufgabe mittels eines Verfahrens mit den Merkmalen des Patentanspruchs 1, mittels einer Vorrichtung mit den Merkmalen des Patentanspruchs 6 und mittels eines Hygieneprodukts mit den Merkmalen des Patentanspruchs 15. Bevorzugte Weiterbildungen der erfinderischen Grundidee sind Gegenstand von Unteransprüchen, deren Wortlaut hiermit durch ausdrückliche Bezugnahme in die Beschreibung aufgenommen wird, um Textwiederholungen nach Möglichkeit zu vermeiden.

Erfindungsgemäß ist ein Verfahren zur Herstellung eines Laminats, welches Laminat eine erste Materialbahn umfasst, vorzugsweise aus einem textilen Flächengebilde, höchst vorzugsweise Vlies- oder Nonwoven-Material, oder aus Folie, welche erste Materialbahn mit Abschnitten wenigstens einer zweiten Materialbahn aus einem elastisch dehnbaren Material, vorzugsweise Schaumstoff, verbunden wird, wobei die erste Materialbahn und die zweite Materialbahn kontinuierlich einer Applikatoreinrichtung zugeführt werden, in welcher Applikatoreinrichtung a) die zweite Materialbahn quer zu ihrer Längserstreckung geschnitten wird, vorzugsweise getaktet, höchst vorzugsweise mittels eines als Messerwalze ausgebildeten ersten Walzenmittels, um die genannten Abschnitte zu erzeugen; b) die genannten Abschnitte bei ihrer Erzeugung in Schritt a) mit einem rotierenden zweiten Walzenmittel in schlüpfendem Reibkontakt stehen und nach erfolgtem Abschneiden von dem zweiten Walzenmittel mitgenommen werden, wobei vorzugsweise das zweite Walzenmittel an seiner Mantelfläche verglichen mit einer Zuführgeschwindigkeit der zweiten Materialbahn mit einer höheren Umfangsgeschwindigkeit rotiert; c) die genannten Abschnitte von dem zweiten Walzenmittel an ein drittes Walzenmittel übergeben werden, auf dessen Mantelfläche die Abschnitte in tangentialer Richtung im Wesentlichen schlupffrei gehalten werden; d) die erste Materialbahn einem vierten Walzenmittel zugeführt wird; e) die genannten Abschnitte an dem vierten Walzenmittel mit Verbindungsbereichen der ersten Materialbahn zusammengeführt werden, wobei das vierte Walzenmittel an seiner Mantelfläche mit einer höheren Umfangsgeschwindigkeit als das dritte Walzenmittel rotiert, so dass die Abschnitte beim Verbinden mit der ersten Materialbahn gedehnt werden, dadurch gekennzeichnet, dass die ersten bis vierten Walzenmittel separat und getrennt voneinander steuerbar motorisch angetrieben werden.

Eine erfindungsgemäße Vorrichtung bzw. Applikatoreinrichtung zur Herstellung eines Laminats, welches Laminat eine erste Materialbahn umfasst, vorzugsweise aus einem textilen Flächengebilde, höchst vorzugsweise Vlies- oder Nonwoven-Material oder aus Folie, welche erste Materialbahn mit Abschnitten wenigstens einer zweiten Materialbahn aus einem elastisch dehnbaren Material, vorzugsweise Schaumstoff, mittels der Applikatoreinrichtung verbindbar ist, welche Applikatoreinrichtung aufweist: a) ein erstes angetriebenes Walzenmittel, welches erstes Walzenmittel dazu ausgebildet ist, die zweite Materialbahn quer zu ihrer Längserstreckung zu schneiden, vorzugsweise getaktet, um die genannten Abschnitte zu erzeugen, welches erste Walzenmittel höchst vorzugsweise als Messerwalze ausgebildet ist; b) ein zweites angetriebenes Walzenmittel, welches zweites Walzenmittel derart mit dem ersten Walzenmittel zusammenwirkend ausgebildet ist, dass die genannten Abschnitte bei ihrer Erzeugung mit dem rotierenden zweiten Walzenmittel in schlüpfendem Reibkontakt stehen und nach erfolgtem Abschneiden von dem zweiten Walzenmittel mitgenommen werden; c) ein drittes angetriebenes Walzenmittel, welches drittes Walzenmittel derart mit dem zweiten Walzenmittel zusammenwirkend ausgebildet ist, dass die genannten Abschnitte von dem zweiten Walzenmittel an das dritte Walzenmittel übergeben werden, dessen Mantelfläche eine Haltestruktur aufweist, welche Haltestruktur dazu ausgebildet ist, die Abschnitte in tangentialer Richtung schlupffrei auf der Mantelfläche zu halten; und d) ein viertes angetriebenes Walzenmittel, welches viertes Walzenmittel zusammen mit dem dritten Walzenmittel dazu ausgebildet ist, die erste Materialbahn und die genannten Abschnitte der zweiten Materialbahn zusammenzuführen; wobei e) das vierte Walzenmittel an seiner Mantelfläche eine höhere Umfangsgeschwindigkeit aufweist als das dritte Walzenmittel, um die Abschnitte beim Aufbringen auf die erste Materialbahn zu dehnen, ist dadurch gekennzeichnet, dass die ersten bis vierten Walzenmittel separat und getrennt voneinander steuerbar motorisch antreibbar sind und separate Walzenmittelantriebe aufweisen.

Die vorliegende Erfindung erreicht die kontinuierliche Herstellung eines Laminats, welches Laminat anschließend zur Herstellung körpergeformter Hygieneprodukte verwendet werden kann, durch das Zusammenwirken von vier Walzenmitteln, welche einen kontinuierlichen Verfahrensverlauf gewährleisten. In diesem Zusammenhang werden die erste Materialbahn und wenigstens eine zweite Materialbahn jeweils kontinuierlich der Applikatoreinrichtung zugeführt. Diese Applikatoreinrichtung ist zunächst dazu ausgebildet, die zweite Materialbahn quer zu ihrer Längserstreckung zu schneiden, um Abschnitte der zweiten Materialbahn zu erzeugen. Vorzugsweise wird dieser Schneidevorgang mittels einer getaktet betriebenen Messerwalze durchgeführt, welche das erste Walzenmittel bildet. Diese Messerwalze weist vorzugsweise - wie alle Walzenmittel der vorliegenden Erfindung - einen eigenen Antrieb auf und kann zudem mehrnutzig ausgebildet sein, was bedeutet, dass über ihren Umfang mehrere Schneidmittel oder Messer verteilt angeordnet sind, vorzugsweise vier Messer in einem Winkelabstand von jeweils 90°.

Die genannten Abschnitte der zweiten Materialbahn stehen bereits vor bzw. bei ihrer Erzeugung im Verfahrensschritt a) mit dem rotierenden zweiten Walzenmittel in schlüpfendem Reibkontakt, so dass sie nach erfolgtem Abschneiden von dem zweiten Walzenmittel mitgenommen werden. Vorzugsweise rotiert dabei das zweite Walzenmittel verglichen mit einer Zuführgeschwindigkeit der zweiten Materialbahn mit einer höheren Umfangsgeschwindigkeit, was einerseits zu dem bereits erwähnten schlüpfenden Reibkontakt führt, und andererseits bereits eine gewisse Vordehnung der Materialbahnabschnitte bewirkt. In diesem Zusammenhang kann vorgesehen sein, dass das zweite Walzenmittel als Saugwalze ausgebildet ist und entsprechend an seiner Mantelfläche Durchbrüche (Saugöffnungen oder -bohrungen) aufweist, durch welche Durchbrüche Luft von außen nach innen in das zweite Walzenmittel hinein saugbar ist, um die zweite Materialbahn bzw. die Materialbahnabschnitte prozesssicher auf der Umfangsfläche des zweiten Walzenmittels zu halten. Dies ist gleichbedeutend damit, dass an der Mantelfläche des zweiten Walzenmittels im Betrieb ein Unterdruck erzeugt wird, um insbesondere den schlüpfenden Reibkontakt in Verfahrensschritt b) zu gewährleisten.

Es kann vorgesehen sein, dass eine Taktung des ersten Walzenmittels und/oder die Rotationsgeschwindigkeit des zweiten Walzenmittels im Verfahrensschritt a) bzw. im Verfahrensschritt b) derart gewählt werden, dass die zweite Materialbahn jeweils genau zwischen den Durchbrüchen des zweiten Walzenmittels geschnitten wird.

Dies ist gleichbedeutend damit, dass eine Taktung des ersten Walzenmittels und/oder die Rotationsgeschwindigkeit des zweiten Walzenmittels derart gewählt ist bzw. sind, dass die Schneidmittel (Messer) des ersten Walzenmittels jeweils gerade zwischen den Durchbrüchen des zweiten Walzenmittels auf die zweite Materialbahn einwirken. Auf diese Weise ist gewährleistet, dass die zweite Materialbahn bzw. deren Abschnitte vor und nach dem Schneidvorgang sicher auf dem zweiten Walzenmittel gehalten sind. Je nach Einstellung des Unterdrucks kann in diesem Bereich bereits eine gewisse Dehnung der zweiten Materialbahn bzw. der entsprechenden Abschnitte erfolgen. Um in der Praxis zu gewährleisten, dass die zweite Materialbahn immer zwischen den Durchbrüchen (Saugbohrungen) des zweiten Walzenmittels geschnitten wird, kann vorgesehen sein, dass die Rotationsgeschwindigkeit des zweiten Walzenmittels auf ein ganzzahliges Vielfaches der Saugbohrungsteilung gerundet wird, vorzugsweise aufgerundet. Hieraus ergibt sich dann die tatsächliche Rotationsgeschwindigkeit des zweiten Walzenmittels, welche vorzugsweise softwareseitig über eine entsprechende Antriebssteuerung einstellbar ist.

Im Zuge einer entsprechenden Weiterbildung der Erfindung werden die ersten bis vierten Walzenmittel separat und getrennt voneinander steuerbar motorisch angetrieben bzw. weisen vorzugsweise alle Walzenmittel separate Antriebe auf, welche über die genannte Steuereinheit oder Antriebssteuerung separat ansteuerbar und aufeinander abstimmbar sind, um die Dehnung der Materialbahnabschnitte und deren Abstand (auch Taktabstand oder Pitch genannt) in einfacher Weise einzustellen.

Im Zuge einer anderen Weiterbildung des erfindungsgemäßen Verfahrens kann vorgesehen sein, dass die erste Materialbahn in Schritt d) zumindest in Klebebereichen mit einem Klebemittel versehen wird, vorzugsweise mit dem Klebemittel benetzt wird, höchst vorzugsweise flächig und/oder getaktet, wobei die Zuführung zu dem vierten Walzenmittel vor, nach oder während des Aufbringens des Klebemittels erfolgt, und wobei das Klebemittel auf einer dem vierten Walzenmittel abgewandten Seite der ersten Materialbahn aufgebracht wird. Durch entsprechende steuerungstechnische Abstimmung mit den ersten bis vierten Walzenmitteln lässt sich so erreichen, dass das Klebemittel nur in solchen Klebebereichen auf die erste Materialbahn aufgebracht wird, in denen tatsächlich Abschnitte der zweiten Materialbahn aufgeklebt werden sollen, um einerseits Klebemittel zu sparen und andererseits ansprechende Produkteigenschaften zu gewährleisten.

Vorrichtungstechnisch kann entsprechend vorgesehen sein, dass die Klebemittel-Aufbringvorrichtung vor, hinter oder im Bereich der Zuführung der ersten materialbahn zu dem vierten Walzenmittel angeordnet ist.

Das Klebemittel kann aufgesprüht oder mittels sogenannter Kontaktknöpfe (Schlitz, Flächendüse) aufgebracht werden. Alternativ kann die Verbindung zwischen erster und zweiter Materialbahn bzw. den entsprechenden Abschnitten auch durch Verprägen (z.B. mittels Ultraschall) oder in anderer Weise erfolgen.

Weiterhin kann vorgesehen sein, dass quer zu einer Förderrichtung der ersten Materialbahn und/oder zu einer Förderrichtung der Abschnitte der zweiten Materialbahn in der Applikatoreinrichtung eine Schnellverstellung zumindest für die Klebemittel-Aufbringvorrichtung vorgesehen ist, um eine Klebemittelauftragsbreite einzustellen. Durch Vorsehen einer derartigen Schnellverstellung kann eine Anpassung der Vorrichtung an veränderte Produkteigenschaften (Abmessungen) grundsätzlich sogar während des laufenden Betriebs erfolgen. Auf jeden Fall ist jedoch keine zeitaufwändige Umrüstung von Formatteilen erforderlich. Auch andere Verbindungseinrichtungen, z.B. Ultraschall-Prägeeinrichtungen, lassen sich auf diese Weise in ihrer Wirkbreite verstellen.

Im Zuge einer wieder anderen Weiterbildung des erfindungsgemäßen Verfahrens kann vorgesehen sein, der Applikatoreinrichtung eine Mehrzahl separater zweiter Materialbahnen vorzugsweise parallel zuzuführen. Alternativ kann jedoch auch vorgesehen sein, dass vor Verfahrensschritt a) die zweite Materialbahn in Richtung ihrer Längserstreckung getrennt wird, um eine Mehrzahl vorzugsweise paralleler zweiter Materialbahnen zu erzeugen, welche anschließend der Applikatoreinrichtung zugeführt werden. In diesem Zusammenhang kann weiter vorgesehen sein, dass die zweiten Materialbahnen quer zu ihrer jeweiligen Längserstreckung zunächst auf ein gewisses Maß beabstandet und anschließend der Applikatoreinrichtung zugeführt werden.

In entsprechender Weiterbildung der erfindungsgemäßen Vorrichtung kann vorgesehen sein, dass vor dem ersten Walzenmittel ein Längstrennmittel für die zweite Materialbahn angeordnet ist. Dieses Längstrennmittel ist entsprechend dazu ausgebildet, die zweite Materialbahn in Richtung ihrer Längserstreckung zu trennen, wie bereits vorstehend beschrieben. Dem Längstrennmittel ist ein Beabstandungsmittel nachgeschaltet, welches Beabstandungsmittel dazu ausgebildet ist, die bereits erwähnte Beabstandung der zweiten Materialbahn quer zu ihrer jeweiligen Längserstreckung vorzunehmen, bevor die zweiten Materialbahnen der Applikatoreinrichtung zugeführt werden.

Im Zuge einer wieder anderen Weiterbildung des erfindungsgemäßen Verfahrens kann vorgesehen sein, dass die Abschnitte der zweiten Materialbahn nach ihrem Aufkleben auf die erste Materialbahn über einen Teilumfang des vierten Walzenmittels von außen gegen das vierte Walzenmittel bzw. gegen die erste Materialbahn gedrückt werden. Dies kann insbesondere mittels eines mitlaufenden Riemens oder dergleichen geschehen. Eine entsprechende Weiterbildung der erfindungsgemäßen Vorrichtung sieht vor, dass an dem dritten Walzenmittel wenigstens ein Abhebemittel zum Abheben der Abschnitte von der Mantelfläche des dritten Walzenmittels angeordnet ist, bei welchem Abhebemittel es sich beispielsweise um den bereits erwähnten Riemen handeln kann. Das Abhebemittel fungiert vorzugsweise zusätzlich als ein Andrückmittel zum Andrücken der Abschnitte an die erste Materialbahn auf dem vierten Walzenmittel. Dabei wurde bereits ausdrücklich auf die bevorzugte Ausgestaltung des kombinierten Abhebe-/Andrückmittels als Riemen hingewiesen. Zusätzlich oder alternativ kann das Abhebe-/Andrückmittel auch als eines der nachfolgend erwähnten Mittel ausgebildet sein: als von innen aus dem dritten Walzenmittel heraus ragender Nocken oder als unter die Abschnitte der zweiten Materialbahn greifender Kamm. Wenn das genannte Abhebe-/Andrückmittel als mit dem dritten Walzenmittel an dessen Mantelfläche mitlaufender Riemen ausgebildet ist, ist vorzugsweise vorgesehen, dass der Riemen in Laufrichtung hinter seiner Ablösestelle von dem dritten Walzenmittel abschnittsweise über die Mantelfläche des vierten Walzenmittels geführt ist, um das erwähnte Andrücken zu bewirken.

Wie weiter oben bereits detailliert beschrieben, sieht eine bevorzugte Weiterbildung der erfindungsgemäßen Vorrichtung vor, dass das Abhebe-/Andrückmittel als ein mit dem dritten Walzenmittel an dessen Mantelfläche mitlaufender Riemen ausgebildet ist, welcher Riemen in Laufrichtung hinter seiner Ablösestelle von dem dritten Walzenmittel abschnittsweise über die Mantelfläche des vierten Walzenmittels geführt ist. Eine solche Führung des Riemens lässt sich in einfacher Weise durch Vorsehen eines weiteren Rollen-/Umlenkmittels für den Riemen erreichen, worauf in der Figurenbeschreibung noch genauer eingegangen wird.

Das zweite Walzenmittel und das dritte Walzenmittel rotieren an ihren Mantelflächen vorzugsweise mit im Wesentlichen gleicher Umfangsgeschwindigkeit. Um die erste Materialbahn und das Laminat prozesssicher zu führen, sieht eine andere Weiterbildung der erfindungsgemäßen Vorrichtung vor, dass das vierte Walzenmittel als Saugwalze ausgebildet ist.

Wie weiter oben bereits grundsätzlich beschrieben wurde, ist das dritte Walzenmittel derart ausgebildet, dass auf seiner Mantelfläche die Abschnitte der zweiten Materialbahn in tangentialer Richtung weitgehend schlupffrei gehalten werden können. Dies lässt sich insbesondere dadurch erreichen, dass das dritte Walzenmittel als Zahnwalze mit über ihrer Mantelfläche verteilt angeordneten Zähnen ausgebildet ist. Diese Zähne sind vorzugsweise symmetrisch und höchst vorzugsweise dreieckförmig (speziell dreieckig-gleichschenklig) ausgebildet und können einen Zahnflankenwinkel von etwa 60° bzw. 75°, höchst vorzugsweise von etwa 69°, aufweisen. Über die Feinheit der Zahnteilung kann die Materialkreppung im Endprodukt beeinflusst werden.

Um die Haltewirkung auf die Abschnitte der zweiten Materialbahn, welche Haltewirkung eine entscheidende Voraussetzung für die angestrebte Dehnung ist, noch zu erhöhen, kann in Weiterbildung der erfindungsgemäßen Applikatoreinrichtung vorgesehen sein, dass die Zähne zumindest bereichsweise entgegen der Laufrichtung bzw. Förderrichtung der Materialbahnabschnitte nach hinten geneigt sind.

Eine besonders bevorzugte Ausgestaltung der erfindungsgemäßen Applikatoreinrichtung sieht außerdem vor, dass das dritte Walzenmittel einen Walzengrundkörper aufweist, der mit Blechscheiben bestückt ist, die beispielsweise mittels Laserschnitt bearbeitet sein können. Die genannten Blechscheiben weisen über ihren Umfang verteilt Zähne auf, welche Zähne wie weiter oben beschrieben ausgebildet sein können. Vorzugsweise sind die genannten Blechscheiben derart untereinander verschachtelt nebeneinander an dem Grundkörper angeordnet, dass ein Vakuumbereich bis zum äußeren Durchmesser des dritten Walzenmittels herausführbar ist, um das Transportieren der Materialbahnabschnitte durch das dritte Walzenmittel zu begünstigen.

Wie bereits angesprochen wurde, weisen vorzugsweise die ersten bis vierten Walzenmittel separat ansteuerbare Antriebe auf. Zusätzlich können auch die Klebemittel-Aufbringvorrichtung und noch vorhandene Zuführ-/Abführmittel für die erste und zweite Materialbahn entsprechend ansteuerbar sein, um auf diese Weise eine einfache Anpassung an geänderte Produktwerte längs des Laminats, insbesondere Dehnung der Materialbahnabschnitte, Taktabstand zwischen aufeinanderfolgenden Abschnitten (Pitch), Abschnittslänge oder dergleichen, durch Anpassen der Relativgeschwindigkeiten der Walzenmittel einzustellen. Vorzugweise erfolgt eine solche Einstellung softwareseitig an einer (gemeinsamen) Steuereinheit für die Walzenmittel, die Zuführ-/Abführmittel und die Klebemittel-Aufbringvorrichtung bzw. eine andere Verbindungseinrichtung.

Gemäß dem Ansatz "product-by-process" erstreckt sich die vorliegende Erfindung auch auf ein mittels des erfindungsgemäßen Verfahrens hergestelltes Laminat, aus welchem sich anschließend, insbesondere durch entsprechendes Zerschneiden quer zur Laminatlängsrichtung, das erfindungsgemäße Hygieneprodukt herstellen lässt. Im Zuge einer Ergänzung um einen entsprechenden Prozessschritt (Schnitt) wird aus dem erfindungsgemäßen Verfahren zur Herstellung eines Laminats ein entsprechendes Verfahren zur Herstellung eines Hygieneprodukts, vorzugsweise eines körpergeformten Inkontinenzprodukts.

Mittels der erfindungsgemäßen Vorrichtung ist erstmalig ein kontinuierlicher Verfahrensablauf bei der Herstellung eines Laminats möglich, so dass sich der Durchsatz deutlich vergrößern lässt.

Weitere Eigenschaften und Vorteile der vorliegenden Erfindung ergeben sich aus der nachfolgenden Beschreibung von Ausführungsbeispielen anhand der Zeichnung.
- Fig. 1: zeigt schematisch eine erfindungsgemäße Applikatoreinrichtung zur Herstellung eines Laminats und das Laminat bzw. dessen Bestandteile an unterschiedlichen Positionen innerhalb der Applikatoreinrichtung;
- Fig. 2: zeigt einen ersten Ausschnitt aus der Applikatoreinrichtung gemäß Figur 1;
- Fig. 3: zeigt einen zweiten Ausschnitt aus der Applikatoreinrichtung gemäß Figur 1;
- Fig. 4: zeigt einen dritten Ausschnitt aus der Applikatoreinrichtung gemäß Figur 1;
- Fig. 5: zeigt ein weiteres Detail der Applikatoreinrichtung gemäß Figur 1; und
- Fig. 6: zeigt das Laminat an unterschiedlichen Positionen innerhalb der Applikatoreinrichtung gemäß Figur 1 sowie schematisch mit Hilfe der erfindungsgemäßen Applikatoreinrichtung bzw. des erfindungsgemäßen Verfahrens hergestellte Hygieneprodukte.

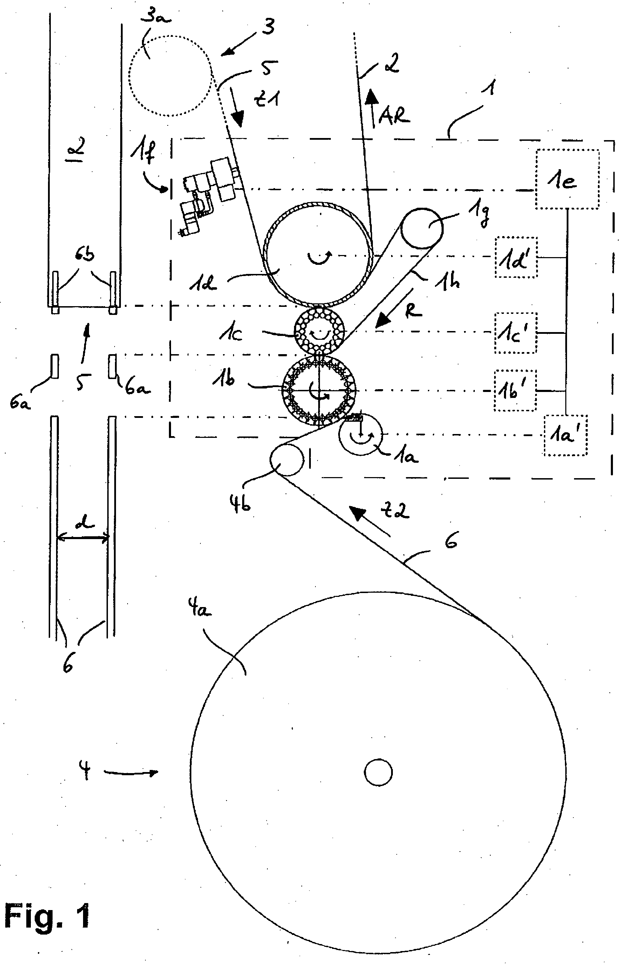

In Figur 1 ist eine Applikatoreinrichtung zur Herstellung eines Laminats schematisch dargestellt, welche Applikatoreinrichtung in ihrer Gesamtheit mit dem Bezugszeichen 1 bezeichnet und mittels einer gestrichelten Box symbolisiert ist. Die Applikatoreinrichtung 1, welche weiter unten detailliert beschrieben wird, wirkt zur Herstellung des Laminats 2 mit Förder- oder Zuführmitteln 3, 4 für eine erste Materialbahn 5 bzw. für eine zweite Materialbahn 6 zusammen. Gemäß Figur 1 sind die Zuführmittel für die erste Materialbahn 5 mit Bezugszeichen 3 bezeichnet, während Bezugszeichen 4 die Zuführmittel für die zweite Materialbahn 6 bezeichnet. Wie sich aus Figur 1 weiterhin ergibt, umfassen die Zuführmittel 4 für die zweite Materialbahn 6 speziell eine Abrollung 4a und eine Vorzugwalze 4b. Bei der zweiten Materialbahn 6 handelt es sich um eine Materialbahn aus einem elastisch dehnbaren Material, vorzugsweise Schaumstoff. Die Zuführmittel 3 für die erste Materialbahn 5 umfassen ebenfalls eine nur angedeutete Abrollung 3a. Bei der ersten Materialbahn 5 handelt es sich um eine Materialbahn aus einem textilen Flächengebilde, beispielsweise einem Vlies- oder Nonwoven-Material. Die Zuführung der ersten Materialbahn 5 bzw. der zweiten Materialbahn 6 zu der Applikatoreinrichtung 1 erfolgt in Richtung der Pfeile Z1 bzw. Z2, jeweils mit einer entsprechenden Zuführgeschwindigkeit.

Es wird nun auf den inneren Aufbau der Applikatoreinrichtung 1 genauer eingegangen:
Die Applikatoreinrichtung umfasst zunächst eine Abfolge von vier Walzenmitteln, welche in Richtung der Zuführung Z2 der zweiten Materialbahn 6 als erstes Walzenmittel 1a, zweites Walzenmittel 1b, drittes Walzenmittel 1c und viertes Walzenmittel 1d bezeichnet sind. Jeweils zwei der genannten ersten bis vierten Walzenmittel 1 a-d sind paarweise zusammenwirkend ausgebildet, worauf weiter unten noch genauer eingegangen wird. Jedes der Walzenmittel 1a-d besitzt seine eigene Antriebsvorrichtung (Motor), welche in Figur 1 entsprechend mit Bezugszeichen 1a'-1d' bezeichnet sind. Strichpunktierte Linien symbolisieren die steuerungstechnische bzw. antriebstechnische Wirkverbindung zwischen den Walzenmitteln 1 a-d und den zugehörigen Antrieben 1a'-d'. Zur gemeinsamen, abgestimmten Steuerung der Antriebe 1a'-d' ist eine Steuerungseinheit vorgesehen, welche in Figur 1 mit Bezugszeichen 1e bezeichnet ist. Des Weiteren umfasst die Applikatoreinrichtung 1 bei Bezugszeichen 1f eine Klebemittel-Aufbringvorrichtung in Form einer Sprüheinheit, welche Klebemittel-Aufbringvorrichtung 1f ebenfalls in signal- bzw. steuerungstechnischer Wirkverbindung mit der Steuereinheit 1e steht (strichpunktierte Linie in Figur 1). Des Weiteren besitzt die Applikatoreinrichtung 1 gemäß Figur 1 bei Bezugszeichen 1g noch ein Umlenk- oder Rollenmittel für einen Riemen 1 h, welcher Riemen 1 h in Richtung des Pfeils R über das dritte Walzenmittel 1c sowie über einen Teil des Umfangs des vierten Walzenmittel 1 d geführt ist und als Anhebe-/Andrückmittel fungiert, worauf weiter unten noch genauer eingegangen wird.

Weiterhin bezeichnet der Pfeil AR in Figur 1 die Abführrichtung des hergestellten Laminats 2, während die im Bereich der Walzenmittel 1a-1d eingezeichneten Pfeile die Rotationsrichtung des jeweiligen Walzenmittels angeben, welche Rotation jeweils durch den zugehörigen Antrieb 1a'-1d' bewirkt ist.

Im linken Teil von Figur 1 ist in Draufsicht das fertige Laminat 2 bzw. die erste Materialbahn 5, die zweite Materialbahn 6 und Abschnitte 6a der zweiten Materialbahn 6 in unterschiedlichen Bearbeitungszuständen innerhalb der Applikatoreinrichtung 1 dargestellt. Die nach links aus der Applikatorvorrichtung 1 herausgeführten strichpunktierten Linien sollen verdeutlichen, in welchen Positionen innerhalb der Applikatoreinrichtung 1 ein bestimmter Bearbeitungszustand gerade erreicht ist.

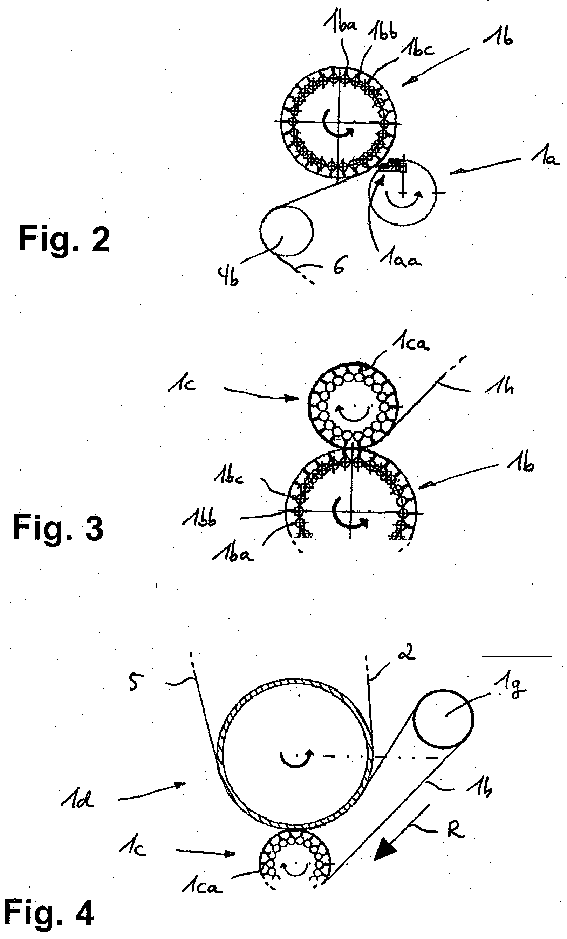

Die Ausschnitte gemäß den Figuren 2 bis 5 stellen bestimmte Bereiche innerhalb der Applikatoreinrichtung 1 vergrößert dar und sollen im Folgenden dazu dienen, die Funktionsweise der Applikatoreinrichtung 1 genauer zu erläutern. Dazu wird im Folgenden neben Figur 1 auch auf die Detaildarstellungen gemäß den Figuren 2 bis 5 explizit Bezug genommen. Aus Gründen der Übersichtlichkeit sind dabei bestimmte Bezugszeichen nur in den Ausschnittsvergrößerungen gemäß Figur 2 bis 5 eingezeichnet.

Die zweite Materialbahn 6 wird von der Abrollung 4a in Richtung des Pfeils Z2 über die Vorzugwalze 4b mit einer bestimmten Zuführgeschwindigkeit der Applikatoreinrichtung 1 im Bereich des ersten Walzenmittels 1 a und des zweiten Walzenmittels 1b zugeführt, wie insbesondere der Figur 2 zu entnehmen ist. Das zweite Walzenmittel 1b ist als Saug- oder Vakuumwalze ausgebildet, so dass die zweite Materialbahn 6 prozesssicher auf der Mantel- bzw. Umfangsfläche des zweiten Walzenmittels 1 b anliegt. Das zweite Walzenmittel 1 b weist zu diesem Zweck an seiner Mantelfläche Durchbrüche oder Saugöffnungen 1ba, 1bb, 1bc,... auf, von denen aus Gründen der Übersichtlichkeit in Figur 2 nur einige wenige explizit bezeichnet sind. Darüber hinaus dürfte die grundsätzliche Ausgestaltung einer Vakuum-/Saugwalze dem Fachmann hinreichend bekannt sein. Das in Figur 2 ebenfalls vergrößert dargestellte erste Walzenmittel 1a wirkt mit dem zweiten Walzenmittel 1 b bzw. der zweiten Materialbahn 6 wie folgt zusammen: am äußeren Umfang des ersten Walzenmittels 1 a ist gemäß Figur 2 ein Schneidmittel in Form eines Messers 1aa angeordnet, welches bei Drehung des ersten Walzenmittels 1a dazu ausgebildet und eingerichtet ist, die zweite Materialbahn 6 auf der Umfangsfläche des zweiten Walzenmittels 1b zu durchtrennen. Während das zweite Walzenmittel 1 b kontinuierlich rotiert, rotiert das erste Walzenmittel 1 a getaktet, so dass Abschnitte 6a der zweiten Materialbahn 6 erzeugt werden, deren Länge insbesondere von der Zuführgeschwindigkeit der zweiten Materialbahn 6 und der Taktung des ersten Walzenmittels 1 a abhängt. Das erste Walzenmittel 1 a wird aufgrund seiner speziellen Ausbildung auch als Messerwalze bezeichnet.

Das zweite Walzenmittel 1 b rotiert vorzugsweise mit einer solchen Geschwindigkeit, dass seine Umfangsgeschwindigkeit höher ist als die Zufuhrgeschwindigkeit der zweiten Materialbahn 6. Auf diese Weise befindet sich die zweite Materialbahn 6 vor und insbesondere bei Erzeugung der genannten Abschnitte 6a in einem schlüpfenden Reibkontakt mit dem zweiten Walzenmittel 1b und wird anschließend, d.h. nach Erzeugung der genannten Abschnitte 6a durch Einwirken der Messerwalze 1 a von dem zweiten Walzenmittel 1 b entsprechend beschleunigt.

Die Rotations- bzw. Umfangsgeschwindigkeit des zweiten Walzenmittels 1b ist derart auf die Taktung des ersten Walzenmittels bzw. der Messerwalze 1 a abgestimmt, dass der Schnitt quer zur Längserstreckung der zweiten Materialbahn 6 immer zwischen zwei Saugöffnungen 1ba, 1 bb, 1 bc,... erfolgt.

Unter Bezugnahme auf Figur 1 sind die mit Hilfe des ersten Walzenmittels 1a erzeugten Abschnitte der zweiten Materialbahn 6 im linken Teil der Figur mit Bezugszeichen 6a bezeichnet. Es sei in diesem Zusammenhang darauf hingewiesen, dass in Figur 1 zwei zweite Materialbahnen 6 mit parallelem Verlauf dargestellt sind, ohne dass die Erfindung auf eine solche Anzahl zweiter Materialbahnen 6 beschränkt wäre. In diesem Zusammenhang ist es grundsätzlich möglich, für die beiden zweiten Materialbahnen 6 jeweils getrennte Abrollungen 4a und/oder Vorzugwalzen 4b vorzusehen, um somit zwei vollständig separate zweite Materialbahnen der Applikatoreinrichtung 1 zuzuführen, wie weiter oben anhand von Figur 2 bereits detailliert beschrieben. Es ist jedoch auch möglich, anstelle von zwei separaten Materialbahnen 6 nur eine einzige zweite Materialbahn 6 vorzusehen, welche vor ihrer Zuführung zu den ersten und zweiten Walzenmitteln 1a, 1b parallel zu ihrer Längserstreckung vorzugsweise mittig getrennt wird, um zwei oder mehr parallele zweite Materialbahnen 6 herzustellen. Dies geschieht mittels eines in Figur 1 nicht explizit dargestellten Längstrennmittels, welchem ein Beabstandungsmittel nachzuschalten ist, um die erzeugten parallelen zweiten Materialbahnen 6 auf den links in Figur 1 dargestellten Abstand d zueinander zu bringen.

Die genannten Abschnitte 6a der zweiten Materialbahn 6 werden auf der Umfangsfläche des zweiten Walzenmittels 1 b mitgeführt und anschließend gemäß Figur 3 an das dritte Walzenmittel 1c übergeben. Dabei ist die Rotationsgeschwindigkeit des zweiten Walzenmittels 1 b und des dritten Walzenmittels 1 c derart aufeinander abgestimmt, dass die genannten Walzenmittel 1 b, 1 c mit im Wesentlichen gleicher Umfangsgeschwindigkeit rotieren. Das dritte Walzenmittel 1 c ist ebenfalls nach Art einer Saug-/Vakuumwalze ausgebildet, um die Abschnitte 6a der zweiten Materialbahn 6 auf seiner Umfangsfläche zu halten. Zu diesem Zweck weist das dritte Walzenmittel 1c entsprechende Saugöffnungen oder -durchbrüche auf, von denen in Figur 3 bei Bezugszeichen 1 ca nur eine bzw. einer explizit bezeichnet ist.

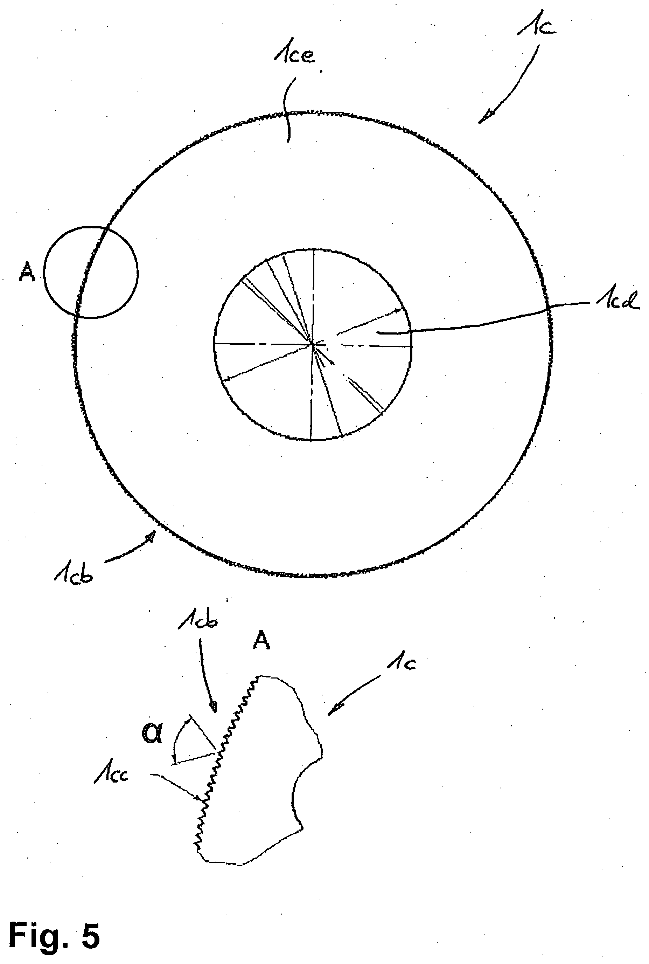

Wie der Abbildung in Figur 5 zusätzlich noch zu entnehmen ist, weist das dritte Walzenmittel 1c an seiner Mantel- bzw. Umfangsfläche eine Haltestruktur 1cb auf, welche Haltestruktur 1cb dazu ausgebildet ist, die bereits erwähnten Abschnitte 6a der zweiten Materialbahn 6 in tangentialer Richtung bezogen auf die Mantelfläche des dritten Walzenmittels 1c schlupffrei zu halten. Dies ist insbesondere anhand einer Ausschnittsvergrößerung "A" in Figur 5 genauer dargestellt. Demnach ist die erwähnte Haltestruktur 1cb gebildet aus einer Abfolge von über die Mantelfläche des dritten Walzenmittels 1c verteilt angeordneten Zähnen 1 cc, welche Zähne 1 cc dreieckig-gleichschenklig ausgebildet sind. Ein entsprechender Zahnflankenwinkel ist in Figur 5 mit Bezugszeichen α bezeichnet und beträgt vorzugsweise etwa 69°.

Eine zeichnerisch nicht dargestellte Abwandlung der vorstehend beschriebenen Haltestruktur 1cb sieht vor, dass die Zähne 1cc entgegen der Laufrichtung der zweiten Materialbahn 6 bzw. der Abschnitte 6a nach hinten geneigt sind, um die angesprochene tangentiale Haltewirkung zu verbessern, wenn die Abschnitte von dem vierten Walzenmittel 1d mit erhöhter Geschwindigkeit übernommen werden.

Eine besondere Ausgestaltung des dritten Walzenmittels 1c sieht vor, dass dieses aus einem in Figur 5 mit Bezugszeichen 1cd bezeichneten Walzengrundkörper gebildet ist, welcher Walzengrundkörper 1cd mit einer Anzahl von Blechscheiben 1 ce bestückt ist, welche Blechscheiben 1 ce über ihren Umfang verteilt die bereits angesprochenen Zähne 1cc aufweisen. Die Blechscheiben 1ce sind dabei vorzugsweise derart untereinander verschachtelt nacheinander an den Grundkörper 1cd angeordnet, dass ausgehend vom Walzengrundkörper 1cd ein Vakuumbereich bis zum äußeren Durchmesser, d.h. bis in den Bereich der Zähne 1cc des dritten Walzenmittels 1c herausführbar ist.

Bezug nehmend auf Figur 4 gelangen die Abschnitte 6a der zweiten Materialbahn 6 anschließend in den Bereich der Mantel- bzw. Umfangsfläche des vierten Walzenmittels 1d, welches vierte Walzenmittel 1d vorliegend auch als Arbeitstrommel bezeichnet wird und zunächst als Führung für die erste Materialbahn dient. Auch das vierte Walzenmittel bzw. die Arbeitstrommel 1d kann als Saugoder Vakuumtrommel ausgebildet sein, um die Führung der ersten Materialbahn 5 bzw. des Laminats 2 prozesssicher zu gestalten (vgl. Figur 1).

Das dritte Walzenmittel 1c übergibt die Abschnitte 6a der zweiten Materialbahn 6 an die mit größerer Umfangsgeschwindigkeit rotierende Arbeitstrommel 1d, welche die genannten Abschnitte 6a zusammen mit der ersten Materialbahn 5 auf ihrer Umfangsfläche mitführt, wobei das Material der Abschnitte 6a gedehnt wird, da es durch die Haltestruktur 1 cb an dem dritten Walzenmittel 1 c in tangentialer Richtung schlupffrei gehalten ist. Dies ist in Figur 1 (links) bei Bezugszeichen 6b dargestellt, welches die bereits teilweise gedehnten Abschnitte der zweiten Materialbahn 6 bezeichnet. In diesem Zusammenhang werden die genannten Abschnitte 6a, 6b der zweiten Materialbahn 6 auf die Außenseite der ersten Materialbahn 5 aufgebracht, welche ebenfalls um die Arbeitstrommel 1d geführt ist, wie insbesondere in Figur 4 dargestellt. Vor dem Aufbringen der genannten Abschnitte 6a, 6b der zweiten Materialbahn 6 auf die erste Materialbahn 5 wurde Letztere mittels der Klebemittel-Aufbringvorrichtung 1f zumindest bereichsweise in Klebebereichen mit einem Klebemittel versehen, um die erste Materialbahn 5 und die Abschnitte 6a, 6b der zweiten Materialbahn 6 dauerhaft zu dem Laminat 2 zu verbinden.

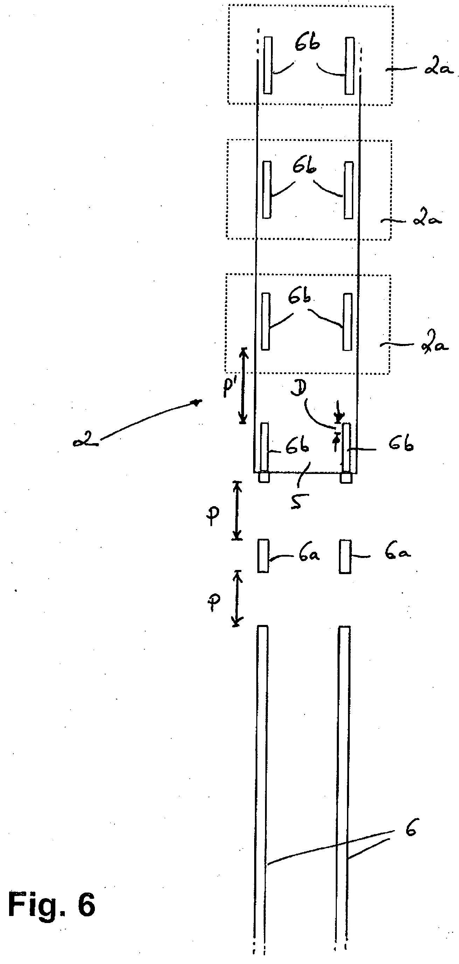

Um eine sichere Klebeverbindung zwischen der ersten Materialbahn 5 und den genannten Abschnitten 6a, 6b der zweiten Materialbahn 6 zu gewährleisten, sind das bereits erwähnte Rollen-/Umlenkmittel 1g und der Riemen 1h vorgesehen. Der Riemen 1 h ist ausgehend von dem Rollen-/Umlenkmittel 1 g in einer umfänglichen Vertiefung in der Mantelfläche des dritten Walzenmittels 1 c geführt, so dass die genannten Abschnitte 6a der zweiten Materialbahn 6 in radialer Richtung außerhalb bzw. oberhalb des Riemens 1 h auf dem dritten Walzenmittel 1c angeordnet sind. Wie insbesondere in Figur 4 gut zu erkennen ist, wird der Riemen 1 h hinter der umfänglichen Berührungsstelle von drittem Walzenmittel 1c und viertem Walzenmittel 1d teilumfänglich über die Umfangsfläche des vierten Walzenmittels 1d geführt, d.h. der Riemen entfernt sich zunehmend von der Umfangsfläche des dritten Walzenmittels 1 c. Er verlässt dabei die bereits angesprochene Vertiefung, wodurch zunächst die Abschnitte 6a, 6b der zweiten Materialbahn 6 von dem dritten Walzenmittel 1c abgehoben und im weiteren Verlauf gegen die Umfangsfläche des dritten Walzenmittels bzw. die darauf befindliche erste Materialbahn 5 gedrückt werden, um das Laminat 2 zu erzeugen. Das fertige Laminat 2 verlässt die Applikatoreinrichtung 1 in Richtung des Pfeils AR und kann dort zur weiteren Verarbeitung aufgewickelt oder direkt zu erfindungsgemäßen Hygieneprodukten weiterbearbeitet werden. Dies ist in Figur 6 abschließend symbolisch dargestellt.

Entsprechend der Anzahl parallel geführter zweiter Materialbahnen 6 bzw. Abschnitte 6a, 6b können mehrere Riemen 1 h vorgesehen sein. Die Riemenbreite ist vorzugsweise kleiner als die Breite der Abschnitte; gleiches gilt für die umfängliche Vertiefung an dem dritten Walzenmittel.

Gezeigt sind in Figur 6 wiederum zwei parallele zweite Materialbahnen 6, welche entweder von separaten Abrollungen zugeführt oder durch Verwendung eines Längstrennmittels während des Herstellungsprozesses erzeugt werden können. Bezugszeichen 6a bezeichnet wiederum die vorstehend beschriebenen Abschnitte der zweiten Materialbahn 6, während Bezugszeichen 6b die gedehnten und zur Erzeugung des Laminats 2 auf die erste Materialbahn 5 aufgeklebten Abschnitte 6a der zweiten Materialbahn 6 bezeichnet. Des Weiteren bezeichnet in Figur 6 das Bezugszeichen P einen sogenannten Taktabstand oder "Pitch" zwischen aufeinanderfolgenden Materialbahnabschnitten 6a, während Bezugszeichen D die Dehnung des aufgeklebten Materialbahnabschnitts 6b gegenüber dem im Wesentlichen ungedehnten Materialbahnabschnitt 6a angibt. Der Materialbahnabschnitt 6a ist nur "im Wesentlichen" ungedehnt, da aufgrund der gewählten Rotationsgeschwindigkeit des zweiten Walzenmittels 1b bereits in diesem Bereich eine gewisse Vordehnung der zweiten Materialbahn 6 erfolgen kann, wie oben beschrieben.

Wie in Figur 6 durch die gestrichelten Rahmenlinien im oberen Bereich der Darstellung symbolisch angedeutet, lassen sich aus dem Laminat 2 Abschnitte 2a erzeugen, welche zur Herstellung erfindungsgemäßer Hygieneprodukte oder dergleichen weiterverarbeitet werden können oder bereits derartige Hygieneprodukte darstellen. Bei den genannten Hygieneprodukten handelt es sich vorzugsweise um körpergeformte Inkontinenzprodukte, wobei die "körpergeformte" Ausbildung dadurch erreicht wird, dass aufgrund des Aufklebens der Materialbahnabschnitte 6a, 6b in einem gedehnten Zustand eine sogenannte Kreppung des Endprodukts bzw. des Laminats 2 oder der Abschnitte 2a resultiert, wie sie beispielsweise auch von Babywindeln her bekannt ist.

Pitch P und Dehnung D lassen sich vorliegend in einfacher Weise auch während des Betriebs durch steuerungstechnische Einwirkung auf die Antriebe 1a'-d' beeinflussen. Der Endabstand der aufgeklebten Materialbahnabschnitte 6b im Produkt (Bezugszeichen P' in Figur 6) ergibt sich aus dem bereits beschriebenen Pitch P aufgrund der zusätzlichen Geschwindigkeitsdifferenz zwischen dem dritten Walzenmittel 1 c und dem vierten Walzenmittel (Arbeitstrommel) 1 d.

Abweichend von der Darstellung, insbesondere in Figur 1 und Figur 2 kann die Messerwalze 1 a (erstes Walzenmittel) auch mehrnutzig ausgebildet sein. Dies bedeutet, dass über den Umfang des ersten Walzenmittels 1a verteilt mehrere Messer 1aa (vgl. Figur 2) angeordnet sein können, vorzugsweise vier Messer in einem Winkelabstand von jeweils 90°.

## Patentansprüche

1. Verfahren zur Herstellung eines Laminats (2), welches Laminat eine erste Materialbahn (5) umfasst, vorzugsweise aus einem textilen Flächengebilde, höchst vorzugsweise Vlies- oder Nonwoven-Material, oder aus Folie, welche erste Materialbahn mit Abschnitten (6a, 6b) wenigstens einer zweiten Materialbahn (6) aus einem elastisch dehnbaren Material, vorzugsweise Schaumstoff, verbunden wird, wobei die erste Materialbahn (5) und die zweite Materialbahn (6) kontinuierlich einer Applikatoreinrichtung (1) zugeführt werden, in welcher Applikatoreinrichtung
a) die zweite Materialbahn quer zu ihrer Längserstreckung geschnitten wird, vorzugsweise getaktet, höchst vorzugsweise mittels eines als Messerwalze ausgebildeten ersten Walzenmittels (1a), um die genannten Abschnitte (6a) zu erzeugen;
b) die genannten Abschnitte bei ihrer Erzeugung in Schritt a) mit einem rotierenden zweiten Walzenmittel (1 b) in schlüpfendem Reibkontakt stehen und nach erfolgtem Abschneiden von dem zweiten Walzenmittel mitgenommen werden, wobei vorzugsweise das zweite Walzenmittel an seiner Mantelfläche verglichen mit einer Zuführgeschwindigkeit der zweiten Materialbahn mit einer höheren Umfangsgeschwindigkeit rotiert;
c) die genannten Abschnitte (6a) von dem zweiten Walzenmittel (1b) an ein drittes Walzenmittel (1c) übergeben werden, auf dessen Mantelfläche die Abschnitte in tangentialer Richtung schlupffrei gehalten werden;
d) die erste Materialbahn (5) einem vierten Walzenmittel (1d) zugeführt wird;
e) die genannten Abschnitte (6a) an dem vierten Walzenmittel (1d) mit Verbindungsbereichen der ersten Materialbahn (5) zusammengeführt werden, wobei das vierte Walzenmittel an seiner Mantelfläche mit einer höheren Umfangsgeschwindigkeit als das dritte Walzenmittel (1c) rotiert, so dass die Abschnitte (6a) beim Verbinden mit der ersten Materialbahn (5) gedehnt (6b) werden;
**dadurch gekennzeichnet, dass**
die ersten bis vierten Walzenmittel (1a-d) separat und getrennt voneinander steuerbar motorisch angetrieben werden.

2. Verfahren nach Anspruch 1,
**dadurch gekennzeichnet, dass**
der schlüpfende Reibkontakt in Schritt b) hergestellt wird, indem an der Mantelfläche des zweiten Walzenmittels (1 b) im Betrieb ein Unterdruck erzeugt wird, vorzugsweise indem das zweite Walzenmittel (1b) in seiner Mantelfläche Durchbrüche (1 ba-c) aufweist, durch welche Durchbrüche Luft von außen nach innen in das zweite Walzenmittel (1 b) gesaugt wird, wobei eine Taktung des ersten Walzenmittels (1 a) und/oder die Rotationsgeschwindigkeit des zweiten Walzenmittels (1b) in Schritt a) und Schritt b) derart gewählt werden, dass die zweite Materialbahn (6) jeweils zwischen den Durchbrüchen (1 ba-c) des zweiten Walzenmittels (1 b) geschnitten wird.

3. Verfahren nach Anspruch 1 oder 2,
**dadurch gekennzeichnet, dass**
die erste Materialbahn (5) in Schritt d) zumindest in Klebebereichen mit einem Klebemittel versehen wird, wobei die erste Materialbahn (5) mit dem Klebemittel benetzt wird, vorzugsweise flächig, höchst vorzugsweise getaktet, wobei die Zuführung zu dem vierten Walzenmittel (1d) vor, nach oder während des Aufbringens des Klebemittels erfolgt, und wobei vorzugsweise das Klebemittel auf einer dem vierten Walzenmittel abgewandten Seite der ersten Materialbahn aufgebracht wird..

4. Verfahren nach mindestens einem der Ansprüche 1 bis 3,
**dadurch gekennzeichnet, dass**
vor Schritt a) die zweite Materialbahn (6) in Richtung ihrer Längserstreckung getrennt wird, um eine Mehrzahl vorzugsweise paralleler zweiter Materialbahnen zu erzeugen, oder dass der Applikatoreinrichtung (1) eine Mehrzahl separater zweiter Materialbahnen (6) zugeführt wird, wobei vorzugsweise die zweiten Materialbahnen (6) quer zu ihrer jeweiligen Längserstreckung auf ein gewünschtes Maß (d) beabstandet und der Applikatoreinrichtung (1) zugeführt werden.

5. Verfahren nach mindestens einem der Ansprüche 1 bis 4,
**dadurch gekennzeichnet, dass**
die Abschnitte (6a, 6b) nach dem Aufkleben über einen Teilumfang des vierten Walzenmittels (1d) von außen gegen die erste Materialbahn (5) gedrückt werden, vorzugsweise mittels eines mitlaufenden Riemens (1 h) oder dergleichen.

6. Applikatoreinrichtung (1) zur Herstellung eines Laminats (2), welches Laminat (2) eine erste Materialbahn (5) umfasst, vorzugsweise aus einem textilen Flächengebilde, höchst vorzugsweise Vlies- oder Nonwoven-Material oder aus Folie, welche erste Materialbahn (5) mit Abschnitten (6a, 6b) wenigstens einer zweiten Materialbahn (6) aus einem elastisch dehnbaren Material, vorzugsweise Schaumstoff, mittels der Applikatoreinrichtung (1) verbindbar ist, welche Applikatoreinrichtung (1) aufweist:
a) ein erstes angetriebenes Walzenmittel (1a), welches erstes Walzenmittel dazu ausgebildet ist, die zweite Materialbahn (6) quer zu ihrer Längserstreckung zu schneiden, vorzugsweise getaktet, um die genannten Abschnitte (6a) zu erzeugen, welches erste Walzenmittel höchst vorzugsweise als Messerwalze ausgebildet ist;
b) ein zweites angetriebenes Walzenmittel (1b), welches zweites Walzenmittel derart mit dem ersten Walzenmittel (1a) zusammenwirkend ausgebildet ist, dass die genannten Abschnitte (6a) bei ihrer Erzeugung mit dem rotierenden zweiten Walzenmittel (1b) in schlüpfendem Reibkontakt stehen und nach erfolgtem Abschneiden von dem zweiten Walzenmittel (1 b) mitgenommen werden;
c) ein drittes angetriebenes Walzenmittel (1c), welches drittes Walzenmittel derart mit dem zweiten Walzenmittel (1b) zusammenwirkend ausgebildet ist, dass die genannten Abschnitte (6a) von dem zweiten Walzenmittel an das dritte Walzenmittel übergeben werden, dessen Mantelfläche eine Haltestruktur (1cb) aufweist, weiche Haltestruktur dazu ausgebildet ist, die Abschnitte (6a) in tangentialer Richtung schlupffrei auf der Mantelfläche des dritten Walzenmittels (1c) zu halten; und
d) ein viertes angetriebenes Walzenmittel (1d), welches viertes Walzenmittel zusammen mit dem dritten Walzenmittel (1c) dazu ausgebildet ist, die erste Materialbahn (5) und die genannten Abschnitte (6a) der zweiten Materialbahn (6) zusammenzuführen; wobei
e) das vierte Walzenmittel (1d) an seiner Mantelfläche eine höhere Umfangsgeschwindigkeit aufweist als das dritte Walzenmittel (1c), um die Abschnitte (6a) beim Aufbringen auf die erste Materialbahn (5) zu dehnen (6b);
**dadurch gekennzeichnet, dass**
die ersten bis vierten Walzenmittel (1a-d) separat und getrennt voneinander steuerbar motorisch antreibbar sind und separate Walzenmittelantriebe (1a'-d') aufweisen.

7. Applikatoreinrichtung (1) nach Anspruch 6,
**dadurch gekennzeichnet, dass**
vor dem ersten Walzenmittel (1a) ein Längstrennmittel für die zweite Materialbahn (6) angeordnet ist, welches Längstrennmittel dazu ausgebildet ist, die zweite Materialbahn in Richtung ihrer Längserstreckung zu trennen, um eine Mehrzahl vorzugsweise paralleler zweiter Materialbahnen zu erzeugen, und dass dem Längstrennmittel ein Beabstandungsmittel nachgeschaltet ist, welches Beabstandungsmittel dazu ausgebildet ist, die zweiten Materialbahnen quer zu ihrer jeweiligen Längserstreckung vor dem Zuführen zu der Applikatoreinrichtung auf ein gewünschtes Maß (d) zu beabstanden.

8. Applikatoreinrichtung (1) nach Anspruch 6 oder 7;
**dadurch gekennzeichnet, dass**
das erste Walzenmittel (1a) mehrnutzig ausgebildet ist, vorzugsweise als Messerwalze mit mehreren über den Umfang verteilt angeordneten Messern (1aa), höchst vorzugsweise vier Messern.

9. Applikatoreinrichtung (1) nach mindestens einem der Ansprüche 6 bis 8,
**dadurch gekennzeichnet, dass**
das zweite Walzenmittel (1b) als Saugwalze ausgebildet ist und in seiner Mantelfläche Durchbrüche (1 ba-c) aufweist, durch welche Durchbrüche Luft von außen nach innen in das zweite Walzenmittel hineinsaugbar ist,
wobei vorzugsweise eine Taktung des ersten Walzenmittels (1a) und/oder die Rotationsgeschwindigkeit des zweiten Walzenmittels (1b) derart gewählt ist, dass die Schneidmittel (1 aa) des ersten Walzenmittels jeweils zwischen den Durchbrüchen (1 ba-c) des zweiten Walzenmittels auf die zweite Materialbahn (6) einwirken.

10. Applikatoreinrichtung (1) nach mindestens einem der Ansprüche 6 bis 9,
**dadurch gekennzeichnet, dass**
das dritte Walzenmittel (1c) als Zahnwalze mit über ihre Mantelfläche verteilt angeordneten Zähnen (1cc) ausgebildet ist, wobei vorzugsweise die Zähne symmetrisch, höchst vorzugsweise dreieckförmig, mit einem Zahnflankenwinkel (α) von etwa 60° bis etwa 75°, vorzugsweise etwa 69°, ausgebildet sind, und wobei höchst vorzugsweise die Zähne (1cc) zumindest bereichsweise entgegen der Laufrichtung der Abschnitte (6a) nach hinten geneigt sind.

11. Applikatoreinrichtung (1) nach mindestens einem der Ansprüche 6 bis 10,
**gekennzeichnet durch**
eine Klebemittel-Aufbringvorrichtung (1f), die dazu ausgebildet ist, die erste Materialbahn (5) zumindest in Klebebereichen mit einem Klebemittel zu versehen, vorzugsweise getaktet, wobei die Klebemittel-Aufbringvorrichtung (1f) zum Benetzen der ersten Materialbahn (5) mit dem Klebemittel ausgebildet ist, insbesondere zum Aufsprühen des Klebemittels auf die erste Materialbahn (5), vorzugsweise auf einer dem vierten Walzenmittel (1d) abgewandten Seite der ersten Materialbahn (5), höchst vorzugsweise getaktet.

12. Applikatoreinrichtung (1) nach Anspruch 11,
**dadurch gekennzeichnet, dass**
quer zu einer Förderrichtung der ersten Materialbahn (5) und/oder zu einer Förderrichtung der Abschnitte (6a) der zweiten Materialbahn (6) in der Applikatoreinrichtung (1) eine Schnellverstellung zumindest für die Klebemittel-Aufbringvorrichtung (1f) vorgesehen ist, um eine Klebemittel-Auftragsbreite einzustellen.

13. Applikatoreinrichtung (1) nach mindestens einem der Ansprüche 6 bis 12,
**dadurch gekennzeichnet, dass**
an dem dritten Walzenmittel (1c) wenigstens ein Abhebemittel (1h) zum Abheben der Abschnitte (6a) von der Mantelfläche des dritten Walzenmittels angeordnet ist, welches Abhebemittel vorzugsweise zusätzlich ein Andrückmittel zum Andrücken der Abschnitte an die ersten Materialbahn (5) auf dem vierten Walzenmittel (1d) bildet, wobei vorzugsweise das Abhebe-/Andrückmittel als eines der folgenden Mittel ausgebildet ist: von innen aus dem dritten Walzenmittel herausragender Nocken, unter die Abschnitte greifender Kamm oder mit dem dritten Walzenmittel (1c) an dessen Mantelfläche mitlaufender Riemen (1h), welcher Riemen in Laufrichtung (R) hinter seiner Ablösestelle von dem dritten Walzenmittel (1c) abschnittsweise über die Mantelfläche des vierten Walzenmittels (1d) geführt ist.

14. Applikatoreinrichtung (1) nach mindestens einem der Ansprüche 6 bis 13,
**dadurch gekennzeichnet, dass**
eine Anpassung an geänderte Produktwerte längs des Laminats (2), insbesondere Dehnung (D) der Abschnitte (6a), Taktabstand (P, P') zwischen aufeinanderfolgenden Abschnitten (6a, 6b), Abschnittslänge oder dergleichen, durch Anpassen der Relativgeschwindigkeiten der Walzenmittel (1a-d) einstellbar ist, vorzugsweise softwareseitig im Bereich der Walzenmittelantriebe (1a'-d').

## Claims

1. Method of producing a laminate (2), which laminate comprises a first material web (5), preferably made of a textile sheet material, most preferably nonwoven material, or made of film, which first material web is joined to sections (6a, 6b) of at least one second material web (6) made of an elastically stretchable material, preferably foam material, the first material web (5) and the second material web (6) being fed continuously to an applicator device (1), in which applicator device
a) the second material web is cut transversely with respect to its longitudinal extent, preferably in cycles, most preferably by means of a first roller means (1 a) in the form of a cutter roller, in order to create the said sections (6a);
b) the said sections, while being created in step a), are in slipping frictional contact with a rotating second roller means (1 b) and, once the cutting operation is complete, are taken up by the second roller means, the second roller means preferably rotating at its outer surface at a higher circumferential speed compared with a feed speed of the second material web;
c) the said sections (6a) are transferred from the second roller means (1 b) to a third roller means (1c) on the outer surface of which the sections are held slip-free in the tangential direction;
d) the first material web (5) is fed to a fourth roller means (1 d);
e) on the fourth roller means (1 d) the said sections (6a) are brought together with joining regions of the first material web (5), the fourth roller means rotating at its outer surface at a higher circumferential speed than the third roller means (1 c), so that the sections (6a) are stretched (6b) while being joined to the first material web (5);
**characterised in that**
the first to fourth roller means (1 a-d) are motor-driven separately and so as to be controllable separately from one another.

2. Method according to claim 1,
**characterised in that**
the slipping frictional contact in step b) is produced by generating a reduced pressure at the outer surface of the second roller means (1 b) during operation, preferably as a result of the second roller means (1 b) having openings (1 ba-c) in its outer surface, through which openings air is sucked from outside to inside into the second roller means (1 b), a cycle timing of the first roller means (1 a) and/or the rotational speed of the second roller means (1 b) in step a) and step b) being selected such that the second material web (6) is each time cut between the openings (1 ba-c) of the second roller means (1b).

3. Method according to claim 1 or 2,
**characterised in that**
in step d) the first material web (5) is provided with an adhesive at least in adhesive regions, wherein the first material web (5) is wetted with the adhesive, preferably over areas of its surface, most preferably in cycles, the web being fed to the fourth roller means (1 d) before, after or during the application of the adhesive, and wherein the adhesive is preferably applied to a side of the first material web remote from the fourth roller means.

4. Method according to at least one of claims 1 to 3,
**characterised in that**
prior to step a), the second material web (6) is separated in the direction of its longitudinal extent in order to create a plurality of preferably parallel second material webs, or the applicator device (1) is fed a plurality of separate second material webs (6), the second material webs (6) preferably being spaced apart to a desired dimension (d) transversely with respect to their respective longitudinal extents and fed to the applicator device (1).

5. Method according to at least one of claims 1 to 4,
**characterised in that**
after the adhesive bonding, the sections (6a, 6b) are pressed against the first material web (5) from the outside over part of the circumference of the fourth roller means (1 d), preferably by means of a belt (1 h) revolving therewith or the like.

6. Applicator device (1) for producing a laminate (2), which laminate (2) comprises a first material web (5), preferably made of a textile sheet material, most preferably nonwoven material, or made of film, which first material web (5) is joinable by means of the applicator device (1) to sections (6a, 6b) of at least one second material web (6) made of an elastically stretchable material, preferably foam material, which applicator device (1) has:
a) a first driven roller means (1a), which first roller means is configured for cutting the second material web (6) transversely with respect to its longitudinal extent, preferably in cycles, in order to create the said sections (6a), which first roller means is most preferably in the form of a cutter roller;
b) a second driven roller means (1 b), which second roller means is configured to co-operate with the first roller means (1 a) in such a way that the said sections (6a), while being created, are in slipping frictional contact with the rotating second roller means (1 b) and, once the cutting operation is complete, are taken up by the second roller means (1 b);
c) a third driven roller means (1c), which third roller means is configured to co-operate with the second roller means (1 b) in such a way that the said sections (6a) are transferred from the second roller means to the third roller means, the outer surface of which has a holding structure (1 cb), which holding structure is configured for holding the sections (6a) slip-free in the tangential direction on the outer surface of the third roller means (1 c); and
d) a fourth driven roller means (1d), which fourth roller means, together with the third roller means (1c), is configured to bring together the first material web (5) and the said sections (6a) of the second material web (6); wherein
e) the fourth roller means (1d) has at its outer surface a higher circumferential speed than the third roller means (1c) in order to stretch (6b) the sections (6a) during application to the first material web (5);
**characterised in that**
the first to fourth roller means (1 a-d) are motor-drivable separately and so as to be controllable separately from one another and have separate roller means drives (1 a'- d').

7. Applicator device (1) according to claim 6,
**characterised in that**
upstream of the first roller means (1a) there is arranged a longitudinal separating means for the second material web (6), which longitudinal separating means is configured for separating the second material web in the direction of its longitudinal extent in order to create a plurality of preferably parallel second material webs, and a spacing means is arranged downstream of the longitudinal separating means, which spacing means is configured for spacing the second material webs apart to a desired dimension (d) transversely with respect to their respective longitudinal extents prior to being fed to the applicator device.

8. Applicator device (1) according to claim 6 or 7,
**characterised in that**
the first roller means (1a) is of multi-slit design, preferably in the form of a cutter roller having a plurality of cutters (1aa) distributed over the circumference, most preferably four cutters.

9. Applicator device (1) according to at least one of claims 6 to 8,
**characterised in that**
the second roller means (1 b) is in the form of a suction roller and has openings (1 ba-c) in its outer surface, through which openings air can be sucked from outside to inside into the second roller means, wherein preferably a cycle timing of the first roller means (1 a) and/or the rotational speed of the second roller means (1 b) is selected such that the cutting means (1 aa) of the first roller means each time act on the second material web (6) between the openings (1 ba-c) of the second roller means.

10. Applicator device (1) according to at least one of claims 6 to 9,
**characterised in that**
the third roller means (1c) is in the form of a toothed roller having teeth (1 cc) distributed over its outer surface, wherein the teeth are preferably of symmetrical construction, most preferably triangular construction, with a tooth flank angle (α) of from approximately 60° to approximately 75°, preferably approximately 69°, and wherein most preferably the teeth (1cc) are, at least in some regions, inclined rearwards opposite to the running direction of the sections (6a).

11. Applicator device (1) according to at least one of claims 6 to 10,
**characterised by**
an adhesive application device (1f) which is configured for providing the first material web (5), at least in adhesive regions, with an adhesive, preferably in cycles, wherein the adhesive application device (1f) is configured for wetting the first material web (5) with the adhesive, especially for spraying the adhesive onto the first material web (5), preferably on a side of the first material web (5) remote from the fourth roller means (1 d), most preferably in cycles.

12. Applicator device (1) according to claim 11,
**characterised in that**
transversely with respect to a transport direction of the first material web (5) and/or to a transport direction of the sections (6a) of the second material web (6) in the applicator device (1) there is provided a quick adjustment means, at least for the adhesive application device (1f), for setting an application width for adhesive.

13. Applicator device (1) according to at least one of claims 6 to 12,
**characterised in that**
at the third roller means (1c) there is arranged at least one lifting means (1 h) for lifting the sections (6a) away from the outer surface of the third roller means, which lifting means preferably additionally forms pressing means for pressing the sections against the first material web (5) on the fourth roller means (1d), the lifting/pressing means preferably being in the form of one of the following means: a cam that projects out of the third roller means from the inside, a comb that engages under the sections, or a belt (1h) that revolves with the third roller means (1c) at the outer surface thereof, which belt, after the point at which it detaches from the third roller means (1c) in the running direction (R), is guided over part of the outer surface of the fourth roller means (1 d).

14. Applicator device (1) according to at least one of claims 6 to 13,
**characterised in that**
an adjustment to changes in product values along the laminate (2), especially stretching (D) of the sections (6a), pitch (P, P') between successive sections (6a, 6b), section length or the like, can be effected by adjusting the relative speeds of the roller means (1 a-d), preferably by means of software in the region of the roller means drives (1 a'-d').

## Revendications

1. Procédé de fabrication d'un laminé (2), lequel laminé comprend une première bande de matériau (5), constituée de préférence d'une structure plane textile, d'une manière particulièrement préférée de matériau en nappe ou non-tissé, ou d'un film, laquelle première bande de matériau est assemblée à des tronçons (6a, 6b) d'au moins une deuxième bande de matériau (6) constituée d'un matériau pouvant être élastiquement allongé, de préférence d'un matériau alvéolaire, sachant que la première bande de matériau (5) et la deuxième bande de matériau (6) sont apportées en continu à un dispositif applicateur (1), dispositif applicateur dans lequel
a) la deuxième bande de matériau est coupée transversalement à son étendue longitudinale, de préférence de façon cadencée, d'une manière particulièrement préférée au moyen d'un premier cylindre (1a) réalisé sous forme de cylindre porte-lames, afin de produire les tronçons précités (6a) ;
b) les tronçons précités se trouvent lors de leur production à l'étape a) en contact frottant de glissement avec un deuxième cylindre rotatif (1b) et sont une fois détachés par la coupe entraînés par le deuxième cylindre, sachant de préférence que le deuxième cylindre tourne sur sa surface circonférentielle avec une plus grande vitesse circonférentielle, par rapport à une vitesse d'alimentation de la deuxième bande de matériau ;
c) les tronçons précités (6a) sont transférés du deuxième cylindre (1b) à un troisième cylindre (1c), sur la surface circonférentielle duquel les tronçons sont maintenus sans glissement en direction tangentielle ;
d) la première bande de matériau (5) est apportée à un quatrième cylindre (1d);
e) les tronçons précités (6a) sont, sur le quatrième cylindre (1d), réunis avec des régions d'assemblage de la première bande de matériau (5), sachant que le quatrième cylindre tourne sur sa surface circonférentielle avec une plus grande vitesse circonférentielle que le troisième cylindre (1c), de sorte que les tronçons (6a) sont allongés (6b) lors de l'assemblage avec la première bande de matériau (5) ;
**caractérisé en ce que** les premier à quatrième cylindres (1a-d) sont entraînés par moteur séparément et en pouvant être commandés indépendamment les uns des autres.

2. Procédé selon la revendication 1, **caractérisé en ce que** le contact frottant de glissement à l'étape b) est réalisé par le fait qu'une dépression est produite en fonctionnement sur la surface circonférentielle du deuxième cylindre (1b), de préférence par le fait que le deuxième cylindre (1b) présente dans sa surface circonférentielle des brèches (1ba-c), brèches par lesquelles de l'air est aspiré de l'extérieur vers l'intérieur dans le deuxième cylindre (1b), sachant qu'un cadencement du premier cylindre (1a) et/ou la vitesse de rotation du deuxième cylindre (1b) aux étapes a) et b) sont choisis de telle sorte que la deuxième bande de matériau (6) est respectivement coupée entre les brèches (1ba-c) du deuxième cylindre (1b).

3. Procédé selon la revendication 1 ou 2, **caractérisé en ce que** la première bande de matériau (5) est pourvue à l'étape d) d'un produit adhésif au moins dans des régions de collage, sachant que la première bande de matériau (5) est imprégnée du produit adhésif, de préférence surfaciquement, d'une manière particulièrement préférée de façon cadencée, sachant que l'apport au quatrième cylindre (1d) s'effectue avant, après ou pendant l'application du produit adhésif, et sachant de préférence que le produit adhésif est appliqué sur un côté de la première bande de matériau qui est opposé au quatrième cylindre.

4. Procédé selon au moins une des revendications 1 à 3, **caractérisé en ce que**, avant l'étape a), la deuxième bande de matériau (6) est sectionnée dans la direction de son étendue longitudinale afin de produire une pluralité de deuxièmes bandes de matériau de préférence parallèles, ou **en ce qu'**une pluralité de deuxièmes bandes de matériau séparées (6) sont apportées au dispositif applicateur (1), sachant de préférence que les deuxièmes bandes de matériau (6) sont espacées transversalement à leur étendue longitudinale respective à une dimension souhaitée (d) et sont apportées au dispositif applicateur (1).

5. Procédé selon au moins une des revendications 1 à 4, **caractérisé en ce que** les tronçons (6a, 6b) sont, à la suite de l'encollage, pressés de l'extérieur contre la première bande de matériau (5) sur une circonférence partielle du quatrième cylindre (1d), de préférence au moyen d'une courroie (1h) à fonctionnement couplé ou similaire.

6. Dispositif applicateur (1) pour la fabrication d'un laminé (2), lequel laminé (2) comprend une première bande de matériau (5), constituée de préférence d'une structure plane textile, d'une manière particulièrement préférée de matériau en nappe ou non-tissé, ou d'un film, laquelle première bande de matériau (5) peut être assemblée au moyen du dispositif applicateur (1) à des tronçons (6a, 6b) d'au moins une deuxième bande de matériau (6) constituée d'un matériau pouvant être élastiquement allongé, de préférence d'un matériau alvéolaire, lequel dispositif applicateur (1) présente :
a) un premier cylindre entraîné (1a), lequel premier cylindre est conçu pour couper la deuxième bande de matériau (6) transversalement à son étendue longitudinale, de préférence de façon cadencée, afin de produire les tronçons précités (6a), lequel premier cylindre est réalisé d'une manière particulièrement préférée sous forme de cylindre porte-lames ;
b) un deuxième cylindre entraîné (1b), lequel deuxième cylindre est conçu pour coopérer avec le premier cylindre (1a) de telle sorte que les tronçons précités (6a) se trouvent lors de leur production en contact frottant de glissement avec le deuxième cylindre rotatif (1b) et sont une fois détachés par la coupe entraînés par le deuxième cylindre (1b) ;
c) un troisième cylindre entraîné (1c), lequel troisième cylindre est conçu pour coopérer avec le deuxième cylindre (1b) de telle sorte que les tronçons précités (6a) sont transférés du deuxième cylindre au troisième cylindre, dont la surface circonférentielle présente une structure de maintien (1cb), laquelle structure de maintien est conçue pour maintenir les tronçons (6a) sur la surface circonférentielle du troisième cylindre (1c) sans glissement en direction tangentielle ; et
d) un quatrième cylindre entraîné (1d), lequel quatrième cylindre (1d) est conçu conjointement avec le troisième cylindre (1c) pour réunir la première bande de matériau (5) et les tronçons précités (6a) de la deuxième bande de matériau (6) ;
e) sachant que le quatrième cylindre (1d) présente sur sa surface circonférentielle une plus grande vitesse circonférentielle que le troisième cylindre (1c), afin d'allonger (6b) les tronçons (6a) lors de leur application sur la première bande de matériau (5) ;
**caractérisé en ce que** les premier à quatrième cylindres (1a-d) peuvent être entraînés par moteur séparément et en pouvant être commandés indépendamment les uns des autres, et présentent des entraînements de cylindres séparés (1a'-d').

7. Dispositif applicateur (1) selon la revendication 6, **caractérisé en ce qu'**un moyen de sectionnement longitudinal pour la deuxième bande de matériau (6) est disposé avant le premier cylindre (1a), lequel moyen de sectionnement longitudinal est conçu pour sectionner la deuxième bande de matériau dans la direction de son étendue longitudinale afin de produire une pluralité de deuxièmes bandes de matériau de préférence parallèles, et **en ce qu'**un moyen d'espacement est monté à la suite du moyen de sectionnement longitudinal, lequel moyen d'espacement est conçu pour espacer les deuxièmes bandes de matériau à une dimension souhaitée (d) transversalement à leur étendue longitudinale respective avant de les apporter au dispositif applicateur.

8. Dispositif applicateur (1) selon la revendication 6 ou 7, **caractérisé en ce que** le premier cylindre (1a) est conçu à fonctionnement multiple, de préférence sous forme de cylindre porte-lames avec plusieurs lames (1aa) disposées en étant réparties sur la circonférence, d'une manière particulièrement préférée quatre lames.

9. Dispositif applicateur (1) selon au moins une des revendications 6 à 8, **caractérisé en ce que** le deuxième cylindre (1b) est réalisé sous forme de cylindre aspirant et présente des brèches (1ba-c) dans sa surface circonférentielle, brèches par lesquelles de l'air peut être aspiré de l'extérieur vers l'intérieur à l'intérieur du deuxième cylindre, sachant de préférence qu'un cadencement du premier cylindre (1a) et/ou la vitesse de rotation du deuxième cylindre (1b) sont choisis de telle sorte que les moyens de coupe (1aa) du premier cylindre agissent respectivement sur la deuxième bande de matériau (6) entre les brèches (1ba-c) du deuxième cylindre.

10. Dispositif applicateur (1) selon au moins une des revendications 6 à 9, **caractérisé en ce que** le troisième cylindre (1c) est réalisé sous forme de cylindre denté avec des dents (1cc) disposées en étant réparties sur sa surface circonférentielle, sachant de préférence que les dents sont réalisées symétriques, d'une manière particulièrement préférée triangulaires, avec un angle de flanc de dent (α) d'environ 60° à environ 75°, de préférence d'environ 69°, et sachant d'une manière particulièrement préférée que les dents (1cc) sont au moins sectoriellement inclinées vers l'arrière à l'encontre du sens de déplacement des tronçons (6a).

11. Dispositif applicateur (1) selon au moins une des revendications 6 à 10, **caractérisé par** un dispositif (1f) d'application de produit adhésif qui est conçu pour pourvoir la première bande de matériau (5) d'un produit adhésif au moins dans des régions de collage, de préférence de façon cadencée, sachant que le dispositif (1f) d'application de produit adhésif est conçu pour imprégner la première bande de matériau (5) du produit adhésif, en particulier pour pulvériser le produit adhésif sur la première bande de matériau (5), de préférence sur un côté de la première bande de matériau (5) qui est opposé au quatrième cylindre (1d), d'une manière particulièrement préférée de façon cadencée.

12. Dispositif applicateur (1) selon la revendication 11, **caractérisé en ce qu'**un réglage rapide au moins pour le dispositif (1f) d'application de produit adhésif, afin de régler une largeur d'application de produit adhésif, est prévu dans le dispositif applicateur (1) transversalement à une direction de transport de la première bande de matériau (5) et/ou à une direction de transport des tronçons (6a) de la deuxième bande de matériau (6).

13. Dispositif applicateur (1) selon au moins une des revendications 6 à 12, **caractérisé en ce qu'**au moins un moyen de dégagement (1h) est prévu sur le troisième cylindre (1c) pour dégager les tronçons (6a) de la surface circonférentielle du troisième cylindre, lequel moyen de dégagement forme de préférence en plus un moyen de pression pour presser les tronçons contre la première bande de matériau (5) sur le quatrième cylindre (1d), sachant de préférence que le moyen de dégagement/pression est réalisé sous la forme d'un des moyens suivants : des ergots dépassant du troisième cylindre depuis l'intérieur, un peigne s'engageant sous les tronçons ou une courroie (1h) à fonctionnement couplé avec le troisième cylindre (1c) sur la surface circonférentielle de ce dernier, laquelle courroie est dirigée sectoriellement sur la surface circonférentielle du quatrième cylindre (1d) après son point de dégagement du troisième cylindre (1c) dans le sens de déplacement (R).

14. Dispositif applicateur (1) selon au moins une des revendications 6 à 13, **caractérisé en ce qu'**une adaptation à des valeurs de produit modifiées le long du laminé (2), en particulier l'allongement (D) des tronçons (6a), l'espacement cadencé (P, P') entre des tronçons successifs (6a, 6b), la longueur de tronçon ou analogues, peut être programmée en adaptant les vitesses relatives des cylindres (1a-d), de préférence sur le plan logiciel au niveau des entraînements de cylindres (1a'-d').
